Europäisches Patentamt

European Patent Office

Office européen des brevets

(11) Veröffentlichungsnummer: **0 510 484 A2**

## EUROPÄISCHE PATENTANMELDUNG

(21) Anmeldenummer: **92106379.8**

(51) Int. Cl.5: **G01N 33/497**

(22) Anmeldetag: **14.04.92**

(30) Priorität: **23.04.91 DE 4113199**

(43) Veröffentlichungstag der Anmeldung:
**28.10.92 Patentblatt 92/44**

(84) Benannte Vertragsstaaten:
**AT BE CH DE DK ES FR GB GR IT LI LU NL SE**

(71) Anmelder: **RETRONIC GmbH**
**Tannenstrasse 2**
**W-7994 Langenargen(DE)**

(72) Erfinder: **Behrendt, Frank**
**Erlachweg 46**
**W-8990 Lindau /B(DE)**

(74) Vertreter: **Riebling, Peter, Dr.-Ing.,**
**Patentanwalt**
**Rennerle 10, Postfach 31 60**
**W-8990 Lindau/B.(DE)**

(54) Verfahren und Vorrichtung zur Ermittlung des Blut-Alkohol-Wertes durch Analyse der Atemluft.

(57) Beschrieben wird ein Verfahren und eine Vorrichtung zur Ermittlung des Blut-Alkohol-Wertes durch Analyse der Atemluft mittels einer Meßapparatur, die in einem Automaten angeordnet ist, wobei die Atemluft durch Einblasen in eine Eingabe einem Sensor zugeführt wird und in Verbindung mit einer elektronischen Analyseermittlung der Wert des Blutalkohols angezeigt wird. Um Manipulationen zu vermeiden und um eine hohe Genauigkeit zu erreichen ist es vorgesehen, daß die Atemluft in genauer Dosierung in stets gleichen Mengen zunächst gespeichert wird und dann dem Sensor mit gleichmäßigem Druck zugeführt wird.

EP 0 510 484 A2

Die Erfindung betrifft ein Verfahren und eine Vorrichtung zur Ermittlung des Blut-Alkohol-Wertes durch Analyse der Atemluft nach dem Oberbegriff des Patentanspruchs 1.

Ein derartiges Verfahren und eine Vorrichtung zur Ermittlung des Blut-Alkohol-Wertes durch Analyse der Atemluft sind bereits bekannt, wobei aber nachteilig der Analyse gewiße Ungenauigkeiten anhaften.

Bei einem bekannten Gerät, welches in Gaststätten und dergleichen aufgestellt wird, ist es vorgesehen, daß der Probant in eine Eingabeöffnung hineinhaucht, wobei der Sensor, welcher die Analyse der Atumluft durchführt, direkt hinter der Eingabeöffnung angeordnet war.

Nachteilig hierbei, es war nur eine geringe Genauigkeit und Empfindlichkeit zu erreichen und außerdem konnten Manipulationen nicht verhindert werden.

Aufgabe der Erfindung ist es deshalb, ein Verfahren und eine Vorrichtung zur Ermittlung des Blut-Alkohol-Wertes der eingangs genannten Art so weiterzubilden, daß Manipulationen verhindert werden und daß höchste Genauigkeiten und Empfindlichkeiten erreicht werden.

Zur Lösung der Aufgabe ist es bei dem Verfahren vorgesehen, daß die Atemluft in genauer Dosierung in stets gleichen Mengen zunächst gespeichert und dann dem Sensor mit gleichmäßigem Druck zugeführt wird.

Die Vorrichtung hierzu sieht vor, daß die Atemluft von der Einblasöffnung über ein Ventil und einen Schlauch zunächst in einen Zylinderraum gelangt, wobei mit dem Einblasen ein Kolben angehoben wird und daß die Atemluft über ein weiteres Ventil mit dem Absinken des Kolbens gleichmäßig über eine Meßleitung dem Sensor zugeführt wird.

Das Wesen der Erfindung liegt demnach darin, daß die Atemluft in besonderer Weise dosiert wird, wobei es vorgesehen ist, daß mittels der Atemluft in einem Zylinderraum ein Kolben angehoben wird, bis schließlich ein bestimmter Füllgrad des Zylinders erreicht wird. Der Kolben befindet sich dann in einer angehobenen Stellung und in dieser Stellung muß die Atemluft für eine Zeitdauer von z. B. 3 Sec. eingeblasen werden. Nach dem Einblasvorgang sinkt der Kolben selbsttätig in dem Zylinderraum ab, wodurch die gespeicherte Atemluft bzw. das Meßgas unter gleichmäßigem Druck kontinuierlich über eine Meßleitung dem Sensor zugeführt wird.

Mit der Verlegung des Sensors von der Einblasöffnung weg zum Ende der Meßapparatur sind zunächst Manipulationen verhindert etwa in der Art, daß der Sensor direkt angehaucht oder angesprüht wird.

Mit der Zuführung des Meßgasen unter stets gleichmäßigem Druck durch das Absinken des Kolbens wird höchste Genauigkeit und Empfindlichkeit der Messung erreicht.

Mit der Dosierung und Zwischenspeicherung der Atemluft und der gleichmäßigen Zuführung zum Sensor werden erhebliche Vorteile erreicht:

Das Meßgas wird in immer gleichem Druck und auch in immer gleichen Mengen an den Sensor rangeführt,

Der Zeitabgriff der zu messenden Luft ist immer stets gleich, wobei die Probanten oder Analyseluft aus der Tiefe der Lunge kommt, Die Luft oder Gaswertigkeit wird bestimmt durch den Eingabedruck den die Lunge beim Test aufbauen muß, wobei stets gleiche wiederholbare Genauigkeiten erreicht werden, weil in einem Zylinderraum ein Kolben um ein bestimtes Maß angehoben wird.

Vorteilhaft ist es vorgesehen, daß die Dosierung einem Zeitabgriff der zu messenden Luft entspricht und daß die über die Dosierung hinausgehende Atemluft in die Umgebungsluft abgeführt wird.

Die entsprechende Dosierung der Atemluft wird innerhalb der Meßapparatur in einem geschlossenen Raum für eine bestimmte Zeitdauer gehalten.

Vorteilhaft ist es auch, daß der Sensor in beheizter Umgebung bei gleichbleibenden Umluftbedingungen angeordnet ist.

In vorteilhafter Ausgestaltung ist es weiterhin vorgesehen, daß bei Erreichen der zur Messung notwendigen Dosierung der Atemluft der Einblasvorgang unterbrochen wird.

Im weiteren ist es zur Erreichung einer hohen Genauigkeit des Meßvorganges vorteilhaft, daß nach jedem Meßvorgang die Luftwege der Meßapparatur zur Reinigung mit Frischluft ausgeblasen werden.

Bei der Vorrichtung zur Ermittlung des Blut-Alkohol-Wertes ist es vorgesehen, daß im Zylinderraum unterhalb des angehobenen Kolbens eine Überlauföffnung in Verbindung mit einer Ausblasleitung angeordnet ist.

In vorteilhafter Ausgestaltung ist weiterhin im Zylinderraum eine IR-Lichtschranke angeordnet, welche die Anhebung des Kolbens erfaßt und das Eingangsventil schließt bzw. den Einblasvorgang unterbricht.

In Verbindung mit der Füllung des Zylinderraums und Anhebung des Kolbens mittels der Atemluft ist es vorgesehen, daß die Atemluft bei angehobenem Kolben und geschlossenen Ein- und Ausgangsventilen innerhalb des Zylinderraumes kurzzeitig gespeichert wird.

Die Atemluft wird im weiteren bei geöffnetem Ausgangsventil über eine Meßleitung mit gleichmäßigem Zustrom dem Sensor zugeführt.

In der Umgebung des Sensors sind vorteilhaft Heizwiderstände und ein Gerätelüfter angeordnet und weiterhin ist es vorgesehen, daß die Meßleitung zum Sensor beheizt ist.

Der Funktionsablauf der Meßanalyse ist vorteilhaft mit Sprachsynthesizern gekoppelt.

Nach dem Eingangsventil ist bei einer vorteilhaften Ausgestaltung eine Luftmembranpumpe angeordnet, welche nach dem Meßvorgang die Eingabe freibläst sowie die Meßapparatur und die Schläuche über einen Luft- und Kondenswasserabfluß ausbläst.

Im Anschluß an die Meßanalyse ist es weiterhin vorgesehen, daß ein Reaktionszeit-Meßgerät gestartet wird, welches der Probant bedienen kann.

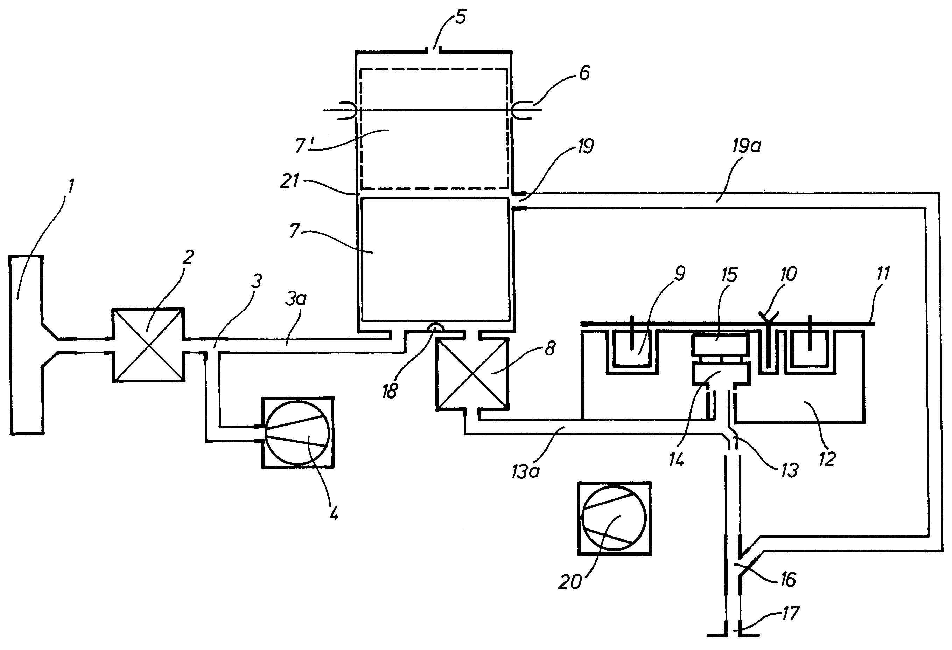

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander. Alle in den Unterlagen - einschließlich der Zusammenfassung - offenbarten Angaben und Merkmale, insbesondere die in der Zeichnung dargestellte räumliche Ausbildung wird als erfindungswesentlich beansprucht, soweit sie einzeln oder in Kombination gegenüber dem Stand der Technik neu sind.

Im folgenden wird die Erfindung anhand einer lediglich einen Ausführungsweg darstellenden Zeichnung näher erläutert. Hierbei gehen aus der Zeichnung und ihrer Beschreibung weitere erfindungswesentliche Merkmale und Vorteile der Erfindung hervor.

Die Zeichnung zeigt das physikalische Analysenmeßprinzip in schematischer Darstellung.

In der Zeichnung ist die Eingabe- bzw. Einblasöffnung 1 erkennbar, über welche der Probant mit einem Strohhalm Luft einbläst, wobei die Eingabe einen Druckkegel aufweist, der in ein Einlaßventil 2 mündet. In Verbindung mit der elektronischen Steuerung über die Münzeingabe öffnet das Einlaßventil 2, wodurch die Atemluft über den Schlauch 3a in den Zylinderraum 21 gelangt, wo ein Kolben 7 zunächst noch in unterster Stellung angeordnet ist.

Mit dem Einblasen der Atemluft wird der Kolben 7 in den Zylinderraum 21 angehoben, und zwar soweit, bis sich der Kolben 7' in angehobener Stellung an der Oberseite des Zylinderraumes 21 befindet. In dieser Stellung unterbricht der Kolben 7' eine Infrarot-Lichtschranke 6, wodurch das Einlaßventil 2 geschlossen wird und im weiteren das Ausgangsventil 8 geöffnet wird.

Der Kolben 7' sinkt nun aus seiner angehobenen Stellung in die untere Stellung, wobei der Kolben 7 die in den Zylinderraum 21 eingeströmte Atemluft kontinuierlich verdrängt und über das Auslaßventil 8 über eine Meßleitung 13 dem Sensor 14 zuführt.

Am Boden des Zylinderraumes 21 befindet sich ein Gummipuffer 18, welcher den Kolben 7 beim Absinken in seine untere Stellung stoppt.

An der Oberseite des Zylinderraumes 21 ist eine Ausgleichsöffnung 5 angeordnet, welche die beim Anheben des Kolbens 7 verdrängte Rstluft im Zylinderraum 21 nach außen abbläst.

Der Sensor 14 ist an einem Sockel 15 angeordnet und im übrigen mit einer elektronischen Regelschaltung verbunden, die in Verbindung mit einem Aluminiumblock 12 auf einer Platine 11 angeordnet ist. Die Platine 11 beinhaltet auch einen Temperaturfühler, um stets gleichbleibende Umluftbedingungen für den Sensor 14 zu gewährleisten. In diesem Zusammenhang sind Heizwiderstände 9 vorgesehen, welche den Sensor und die Meßleitung 13a beheizen. Im weiteren ist ein Gerätelüfter 20 innerhalb des Gerätes in der Nähe des Analyseteils angeordnet, um eine gewiße Umluft herzustellen und dadurch gleichmäßige Umgebungsbedingungen, insbesondere gleiche Temperatur und Luftfeuchtigkeit zu gewährleisten.

Das Meßgas bzw. die Atemluft gelangt von dem Sensor über einen Kondenswasserabfluß 13 und einen Luft- und Kondenswasserabfluß 17 in die Umgebung zurück.

Etwa in der Mitte des Zylinderraumes unterhalb des Kolbens 7' in seiner angehobenen Stellung ist eine Überlauföffnung 19 angeordnet, welche über eine Schlauchleitung 19a und einen Luftverteiler 16 mit dem Luft- und Kondenswasserabfluß 17 in Verbindung steht.

Nachdem der Kolben 7 über die Atemluft angehoben wird befindet sich der Kolben 7' in angehobener Stellung, wodurch nach Erreichen einer bestimmten Zeitdauer, wo die Einblasöffnung 1 betätigt werden muß, die Überlauföffnung 19 von dem Kolben 7' freigegeben wird. Gleichzeitig wird jedoch über die IR-Lichtschranke 6 das Einlaßventil 2 geschlossen und der Meßvorgang wird durch öffnen des Auslaßventiles 8 in Gang gesetzt.

Mit dem Absinken des Kolbens wird die Atemluft komprimiert und in stets gleichen Mengen bei gleichem Druck in kontinuierlichem Zustrom dem Sensor 14 zugeführt, wodurch höchste Empfindlichkeiten und Genauigkeiten erreicht werden.

Nach Beendigung des Meßvorganges ist es vorgesehen, daß über eine Luftmembranpumpe 4 die Einblasöffnung 1, das Ventil 2, sowie die nachfolgend angeordneten Schläuche sowie die gesamte Meßapparatur einschließlich des Sensors 14 mit Umgebungsluft freigeblasen werden.

Im folgenden wird der Funktionsablauf der Analyse beschrieben:
Das Gerät läuft zunächst in einem Testlauf, wobei Schriftzüge für den Alkoholtest sowie Werbebilder und Comicinformationsbilder aufleuchten.

Über einen Münzeinwurf wird das Gerät betätigt, wodurch ein Strohhalm freigegeben wird und erläuternde Schriftzüge für den Alkoholtest aufleuchten.

Im weiteren werden Sprachprozessoren eingeschaltet, welche in Verbindung mit dem Alkoholtest Erläuterungen abgeben und nach Beendigung des Testes Angaben über den Wert des Alkohols machen und eventuell auf einen zusätzlichen Reaktionstest hinweisen.

Der Proband bläst nun mit dem Strohhalm in die Eingabeöffnung 1, wobei die Atemluft über das Einlaßventil in den Zylinderraum 21 gelangt und dort der Kolben 7 angehoben wird, bis die angehobene Stellung des Kolbens 7' erreicht ist.

Das Gerät verhindert Manipulationen und vermittelt eine höchste Genauigkeit und Empfindlichkeit.

Durch den Einsatz des Strohhalmes werden Manipulationen in Verbindung mit dem Einblasen verhindert und im übrigen ist der Sensor entfernt von der Einblasöffnung angeordnet, so daß der Sensor 14 nur über eine Zwischenspeicherung des Atemgases erreichbar ist.

Das Meß- bzw. Atemgas wird zur Analyse in stets gleichem Druck und in gleichen Mengen an den Sensor 14 herangeführt.

Im weiteren ist der Zeitabgriff der zu messenden Luft immer gleich, wobei die Probanten oder Analyseluft aus der Tiefe der Lunge kommt.

Die bestimmte Wertigkeit der Luft oder des Atemgases wird bestimmt durch den Eingabedruck, den die Lunge beim Test aufbaut, wobei durch die Anhebung des Kolbens eine Kontrolle erreicht wird, so daß stets gleiche Zufuhrbedingungen an den Sensor 14 erreicht werden.

Der Sensor 14 ist an einer Aluminium-Meßplatte bzw. Platine 11 angeordnet, die ständig durch Heizwiderstände 9 beheizt wird. Die Platte wird im weiteren über Temperatursensoren 10 auf plus/minus 2° C reguliert. Die Temperatur der Meßplatte beträgt 30° C. Die Zulaufrohre zum Sensor bzw. die Meßleitung 13a ist ebenso wie die Platte des Sensors 14 beheizt, so daß eine Art Verdampfung gewährleistet ist, wobei der Alkoholgehalt in der Atem- bzw. Analyseluft verdampft und somit in besonders gutem Maße den Sensor 14 erreicht.

Um immer den gleichen Meßdruck bzw. das gleiche Volumen zu bekommen, wird das Meßgas in den Zylinderraum 21 eingeblasen, wobei ein bestimmter Druck eine bestimmte Zeit gehalten werden muß, um die Meßanalyse vorzunehmen.

Der Proband bläst bei Freigabe des Einlaßventils 2 in den Zylinderraum 21, wobei der Kolben 7 durch den Luftdruck nach oben gedrückt wird. Hierbei wird die Lichtschranke 6 unterbrochen und die Zeit gemessen.

Fällt der Kolben 7' durch Druckabfall herunter, so schaltet das Gerät automatisch zurück und der Test muß von vorne begonnen werden.

Bleibt der Druck aber über dem Schwellwertzeitpunkt erhalten, so schließt das Einlaßventil 2 und das Ventil 8 öffnet sich. Das vorhandene Meßgas im Zylinderraum 21 wird durch den Kolben 7' komprimiert und durch das Ventil 8 und die Meßleitung 13a zum Sensor 14 geleitet. Das Volumen des Zylinderraumes 21 sowie auch der Druck des Kolbens 7' bleibt ständig gleich, so daß in stets genauer Dosierung bei gleicher Druckströmung der Sensor 14 mit der Atemluft bestrichen wird.

Um zu verhindern, daß sich im Gerät bzw. im Zylinderraum Schwitzwasser ansammelt ist unterhalb des Sensors 14 im Zulauf ein Kondenswasserabfluß 13 angebracht, durch den Kondenswasser ins Freie abgeleitet wird.

Hat der Kolben 7' das Meßgas vollständig aus dem Zylinderraum 21 dem Sensor 14 zugeführt vergeht eine gewiße Zeit (Synchronisationszeit) und das Meßergebnis wird gehalten. Diese Zustände sind beim Test mit 0 % Abweichung immer gleich.

Das Meßsignal wird zweistufig analog umgeformt und dem Digitalteil als auch dem Analogteil zugeführt. Nach dem Test bzw. Halten des Signals setzt die Reinigungsphase des Gerätes ein, d.h., das Ventil 2 bleibt geschlossen, Ventil 8 ist geöffnet und die Membranpumpe 4 fängt an zu arbeiten und bläst von der Eingabe 1 über den Zylinderraum 21 zum Sensor 14 mit 10 bis 15 l Luft alles frei, so daß kein Restalkohol mehr im Gerät verbleibt.

Der Proband muß bei der Betätigung des Gerätes eine gewiße Zeit in das Gerät blasen, bis die Meßauswertung beginnt. Der Kolben 7 wird durch den Luftdruck nach oben gedrückt, wodurch eine Überlauföffnung 19 frei wird, welche ins Freie führt. Nach etwa 3 Sec. während der Proband Atemluft einbläst schließt das Ventil 2 und der Kolben 7' fällt mangels Druck herunter, wobei die komprimierte Luft über das Ventil 8 zum Sensor 14 geleitet wird.

Die Luft oder Meßgasmenge wird demnach durch das Unterbrechen der Lichtschranke 6 bzw. durch das Heraufdrücken des Kolbens 7' in seine obere Stellung bestimmt.

Der Luftdruck, welcher das Atemgas den Sensor 14 zuführt, wird durch das Gewicht des Kolbens 7 bestimmt, wobei stets 0 % Abweichung erreicht werden.

Die Probanten selbst blasen selbstverständlich mit unterschiedlichem Druck in das Gerät, was aber durch die unterschiedliche Flußgeschwindigkeit des Meßgases bzw. der Luft von selbst geregelt wird, insbesondere in Verbindung mit dem Anheben des Kolbens 7 im Zylinderraum 21.

Durch das Anheben des Kolbens 7 ist auch sichergestellt, daß der Proband mindestens 2/3 seiner gesamten Lungenkapazität in das Gerät blasen muß, ehe ein Meßergebnis abgenommen wird.

In Verbindung mit der genauen Dosierung der Atemluft und der Zwischenspeicherung des Atemgases in dem Zylinderraum und der kontrollierten Zuführung zum Sensor 14 werden sehr hohe Genauigkeiten und Empfindlichkeiten erreicht, wobei Manipulationen ausgeschlossen sind.

ZEICHNUNGS-LEGENDE

| 1 | Einblasöffnung |
| 2 | Einlaßventil |
| 3 | Luftverteiler |
| 3a | Schlauch |
| 4 | Luftmembranpumpe |
| 5 | Ausgleichsöffnung |
| 6 | IR-Lichtschranke |
| 7,7' | Kolben |
| 8 | Ausgangsventil |
| 9 | Heizwiderstände |
| 10 | Temperaturfühler |
| 11 | Platine |
| 12 | Aluminiumblock |
| 13 | Kondenswasserabfluß |
| 13a | Meßleitung |
| 14 | Sensor |
| 15 | Sensorsockel |
| 16 | Luftverteiler |
| 17 | Luft- und Kondenswasserabfluß |
| 18 | Gummipuffer |
| 19 | Überlauföffnung |
| 19' | Schlauchleitung |
| 20 | Gerätelüfter |
| 21 | Zylinderraum |

**Patentansprüche**

1. Verfahren zur Ermittlung des Blut-Alkohol-Wertes durch Analyse der Atemluft mittels einer Meßapparatur, die in einem Automat angeordnet ist, der an den Wänden von Gaststätten, Beherbergungsbetrieben und dergleichen aufgehängt werden kann, wobei die Atemluft durch Einblasen in eine Eingabe einem Sensor zugeführt wird, der in Verbindung mit einer elektronischen Analyseermittlung über eine Anzeige den Wert des Blutalkohols digital und analog angibt, **dadurch gekennzeichnet**, daß die Atemluft in genauer Dosierung in stets gleichen Mengen zunächst gespeichert und dann dem Sensor (14) mit gleichmäßigem Druck zugeführt wird.

2. Verfahren nach Anspruch 1, **dadurch gekennzeichnet**, daß die Dosierung einen Zeitabgriff der zu messenden Luft entspricht und daß die über die Dosierung hinausgehende Atemluft in die Umgebungsluft abgeführt wird.

3. Verfahren nach Anspruch 1, **dadurch gekennzeichnet**, daß die entsprechende Dosierung der Atemluft innerhalb der Meßapparatur in einem geschlossenen Raum für eine bestimmte Zeitdauer gehalten wird.

4. Verfahren nach Anspruch 1, **dadurch gekennzeichnet**, daß der Sensor (14) in beheizter Umgebung bei gleichbleibenden Umluftbedingungen angeordnet ist.

5. Verfahren nach Anspruch 1, **dadurch gekennzeichnet**, daß bei Erreichen der zur Messung notwendigen Dosierung der Atemluft der Einblasvorgang unterbrochen wird.

6. Verfahren nach Anspruch 1, **dadurch gekennzeichnet**, daß nch jedem Meßvorgang die Luftwege der Meßapparatur zur Reinigung mit Frischluft ausgeblasen werden.

7. Vorrichtung zur Ermittlung des Blut-Alkohohl-Wertes durch Analyse der Atemluft mittels einer Meßapparatur, die in einem Automat angeordnet ist, der an den Wänden von Gaststätten, Beherbergungsbetrieben und dergleichen aufgehängt werden kann, wobei die Atemluft durch Einblasen in eine Eingabe einem Sensor zugeführt wird, der in Verbindung mit einer elektronischen Analyseermittlung über eine Anzeige den Wert des Blutalkohols digital und analog angibt, **dadurch gekennzeichnet**, daß die Atemluft von der Einblasöffnung (1) über ein Ventil (2) und einen Schlauch (3a) in einen Zylinderraum (21) gelangt, wobei mit dem Einblasen ein Kolben (7) angehoben wird, und daß die Atemluft über ein weiteres Ventil (8) mit dem Absinken des Kolbens (7') gleichmäßig über eine Meßleitung (13a) dem Sensor (14) zugeführt wird.

8. Vorrichtung nach Anspruch 7, **dadurch gekennzeichnet**, daß im Zylinderraum (21) unterhalb des angehobenen Kolbens (7') eine Überlauföffnung (19) in Verbindung mit einer Schlauchleitung (19a) angeordnet ist.

9. Vorrichtung nach Anspruch 7, **dadurch gekennzeichnet**, daß im Zylinderraum (21) eine IR-Lichtschranke (6) angeordnet ist, welche die Anhebung des Kolbens (7) erfaßt und das Ventil (2) schließt bzw. den Einblasvorgang unterbricht.

10. Vorrichtung nach Anspruch 7, **dadurch gekennzeichnet**, daß die Atemluft bei angehobenem Kolben (7') und geschlossenen Ventilen (2,8) innerhalb des Zylinderraumes (21) kurzzeitig gespeichert wird.

**11.** Vorrichtung nach Anspruch 7, **dadurch gekennzeichnet**, daß die Atemluft bei geöffnetem Ventil (8) über eine Meßleitung (13a) mit gleichmäßigem Zustrom dem Sensor (14) zugeführt wird.

**12.** Vorrichtung nach Anspruch 7, **dadurch gekennzeichnet**, daß in der Umgebung des Sensors (14) Heizwiderstände (9) und ein Gerätelüfter (20) angeordnet sind und daß die Meßleitung (13a) zum Sensor (14) beheizt ist.

**13.** Vorrichtung nach Anspruch 7, **dadurch gekennzeichnet**, daß der Funktionsablauf der Meßanalyse mit Sprachprozessoren gekoppelt ist.

**14.** Vorrichtung nach Anspruch 7, **dadurch gekennzeichnet**, daß nach dem Ventil (2) eine Luftmembranpumpe (4) angeordnet ist, welche nach dem Meßvorgang die Eingabe freibläst sowie die Meßapparatur und die Schläuche (3a,13a,19a) über einen Luft- und Kondenswasserabfluß (17) ausbläst.

**15.** Vorrichtung nach Anspruch 7, **dadurch gekennzeichnet**, daß im Anschluß an die Meßanalyse des Blut-Alkohol-Wertes ein Reaktionszeit-Meßgerät gestartet wird.